# EUROPEAN PATENT APPLICATION

(11) **EP 0 928 620 A2**
(43) Date of publication of application: **14.07.1999**
(21) Application number: 98310337.5
(22) Date of filing: 16.12.1998
(51) Int. Cl.: A61M 39/12

(54) **Connector assembly**

(30) Priority: 06.01.1998 GB 9800087
(71) Applicant: Smiths Industries Public Limited Company, London, NW11 8DS (GB)
(72) Inventor: Pagan, Eric, Hythe, Kent CT21 6DN (GB)
(74) Representative: Flint, Jonathan McNeill

(57) **Abstract**

A connector 2, 2', 2'' suitable for a helically-reinforced endotracheal tube 1 has a tapered forward end 20, 20', 20'' with two barbs 23 and 24, 23' and 24', 23'' and 24'' projecting outwardly on opposite sides. The barbs have a rearwardly-inclined forward ramp 25 and a perpendicular rear surface 26 so that the connector can be pushed into the tube but is more difficult to remove. The ends 27 of the barbs taper to provide a smooth continuation with the outside of the connector.

## Description

This invention relates to connectors of the kind having a first end and a second end, the first end being adapted for insertion into an end of a tube for making a sealing fit with the inside of the tube and being tapered at its forward end to facilitate insertion in the tube, and the second end being adapted for connection with a cooperating connector.

Medical connectors may be used to make connection with one end of a tube or tubing. The connector commonly has a tapered end pushed into the machine end of the tube and held in place by the elastic nature of the tube and by friction. The opposite end of the connector may have a tapered male or female fitting, or any other conventional fitting, for making connection to a cooperating connector. Such connectors work satisfactorily in many circumstances. However, in some circumstances, the connector may be liable to be pulled out of the tube inadvertently. It is also difficult to make secure connection to a reinforced tube, such as one reinforced with a helical wire or filament, because the tube will not deform as readily to receive the connector.

It is an object of the present invention to provide an improved connector and an assembly including a connector.

According to one aspect of the present invention there is provided a connector of the above-specified kind, characterised in that the first end has at least one radially-projecting barb on its outer surface, and that the or each barb has a rearwardly-inclined forward ramp surface and a steeper rear surface such that the or each barb enables insertion of the first end into the tube but resists removal of the connector from the tube.

The connector preferably includes two barbs, which may be located diametrically opposite one another. The rear surface of the or each barb is preferably inclined substantially perpendicularly and the ends of the or each barb may be tapered to provide a smooth continuation with the surface of the connector. The first end may comprise a tapered portion at its forward end and a cylindrical portion at its rear end, the or each barb being located on the cylindrical portion. Alternatively, the first end may be tapered and the or each barb may be located on the tapered end. The forward end of the first end may be bevelled to facilitate insertion in the tube.

According to another aspect of the present invention there is provided an assembly of a tube and a connector according to the above one aspect of the invention, the first end of the connector being inserted within an end of the tube.

The tube may be a tracheal tube and it may be reinforced with a helical member.

A tracheal tube assembly including a connector, in accordance with the present invention, will now be described, by way of example, with reference to the accompanying drawings, in which:
- Figure 1: is a side elevation view of the assembly;
- Figure 2: is a side elevation view of the connector to a greater scale;
- Figure 3: is a plan view of the connector of Figure 2;
- Figure 4: is a perspective view of the connector;
- Figure 5: is a plan view of an alternative connector;
- Figure 6: is a side elevation view of another alternative connector;
- Figure 7: is a plan view of the connector of Figure 6; and
- Figure 8: is a perspective view of the connector of Figures 6 and 7.

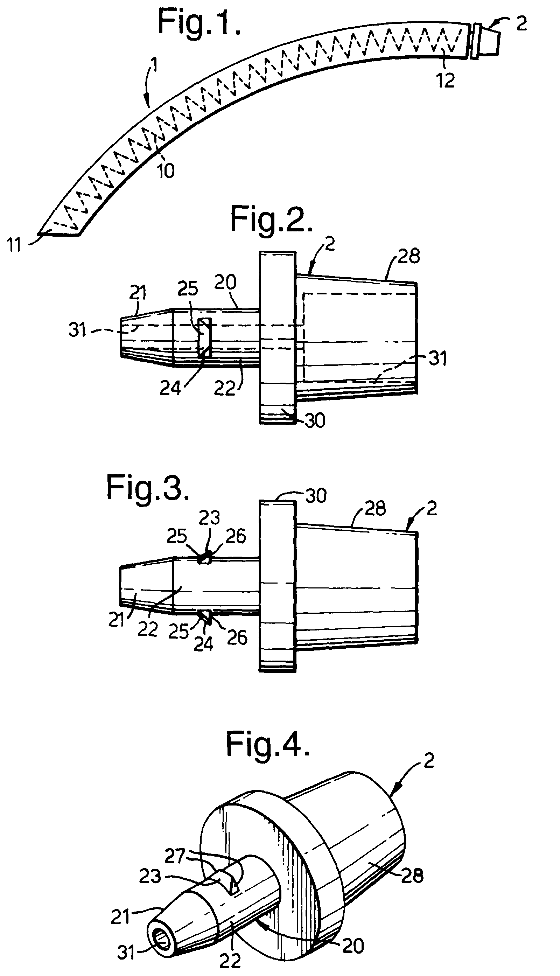

With reference first to Figures 1 to 4, the assembly comprises an endotracheal tube 1 and a connector 2 mounted at the rear or machine end of the tube.

The tube 1 may be entirely conventional and formed of a plastics material such as PVC with an embedded helical reinforcement filament 10 of a harder plastics or metal. The tube 1 is open and bevelled at its forward, patient end 11, and is cut square at its machine end 12.

The connector 2 is moulded from a hard plastics material, such as polyethylene, polyproplyene, styrene, acrylics, SAN or SBS. At its first, patient end, the connector has a tubular spigot 20 of circular section comprising a forward patient end portion 21, which tapers rearwardly to an enlarged diameter, and a rear portion 22 of constant external diameter and cylindrical shape. The external surface of the rear portion 22 is interrupted by two radially-projecting barbs 23 and 24 moulded integrally with the body of the connector and located about midway along the spigot 20. Each barb has an inclined forward surface 25, which slopes outwardly, and a rear surface 26, which is perpendicular. The barbs 23 and 24 extend circumferentially around about one quarter of the circumference of the rear portion 22 and are located diametrically opposite one another. The ends 27 of the barbs 23 and 24 taper to form a substantially smooth continuation of the main part of the surface of the rear portion 22.

At its rear, machine end, the connector 2 has a male tapered portion 28 of increased diameter, which is adapted to be inserted within a conventional 15mm female ventilation coupling (not shown). The rear end portion 28 is separated from the forward spigot 20 by a radially-projecting flange or finger grip 30. A bore 31 extends axially along the length of the connector, opening at opposite ends.

The barbs 23 and 24 grip the inside of the tube 1 to retain it securely on the connector 2. Because of the grip provided by the barbs, the forward end of the connector can have a slightly smaller external diameter than conventional tapered connectors, which rely solely on friction to retain the connector in the tube. This facilitates insertion of the connector 2 in reinforced tubes, which do not expand radially as easily as unreinforced tubes. The forward inclined surfaces 25 of the barbs 23 and 24 allow the connector 2 to be inserted readily into the tube 1; the steeper rear surface 26 resists removal of the connector

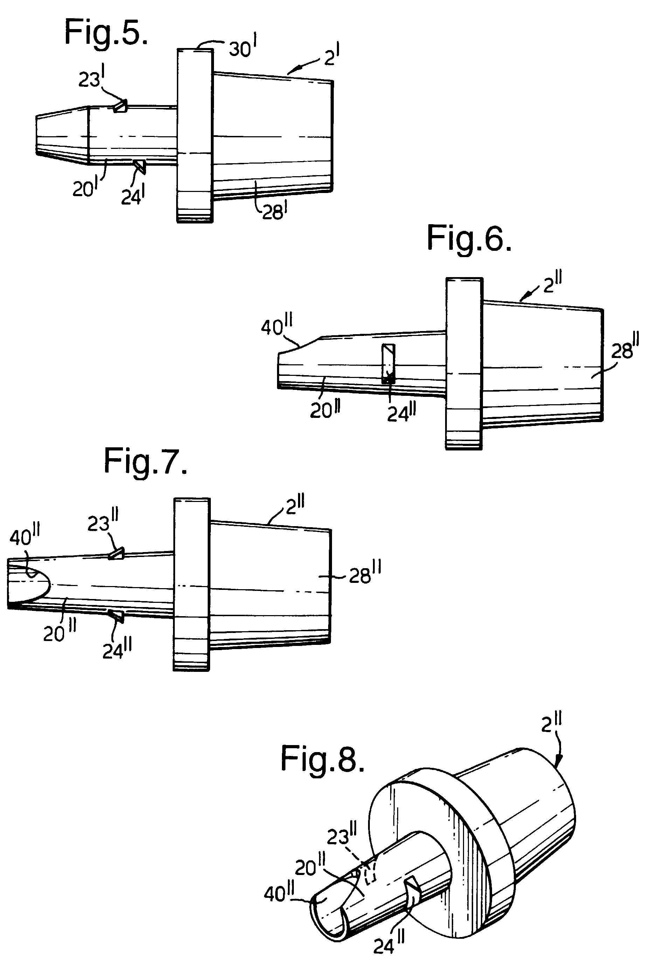

The barbs need not be located at the same point along the spigot but could be offset from one another, as shown in Figure 5, where similar components are given the same numbers as those in the connector shown in Figures 2 to 4, with the addition of a prime '.

Another modification is shown in Figures 6 to 8, where the forward spigot 20'' tapers along its entire length and has a cutaway bevel 40'' at its patient end to ease insertion in the end of the tube.

It will be appreciated that the invention could be used in connectors for various different purposes and with various different tubes.

## Claims

1. A connector (2, 2', 2'') having a first end (20, 20', 20'') and a second end (28, 28', 28''), the first end being adapted for insertion into an end of a tube (1) for making a sealing fit with the inside of the tube and being tapered at its forward end to facilitate insertion in the tube, and the second end (28, 28', 28'') being adapted for connection with a cooperating connector, characterised in that the first end (20, 20', 20'') has at least one radially-projecting barb (23, 24, 23', 24', 23'', 24'') on its outer surface, and that the or each barb has a rearwardly-inclined forward ramp surface (25) and a steeper rear surface (26) such that the or each barb (23, 24, 23', 24', 23'', 24'') enables insertion of the first end (20, 20', 20'') into the tube (1) but resists removal of the connector (2, 2', 2'') from the tube.

2. A connector according to Claim 1 including two barbs (23 and 24, 23' and 24', 23'' and 24'').

3. A connector according to Claim 2, characterised in that the barbs (23 and 24, 23'' and 24'') are located diametrically opposite one another.

4. A connector according to any one of the preceding claims, characterised in that the rear surface (26) of the or each barb (23 and 24, 23' and 24', 23'' and 24'') is inclined substantially perpendicularly.

5. A connector according to any one of the preceding claims, characterised in that the ends (27) of the or each barb (23, 24, 23', 24', 23'', 24'') are tapered to provide a smooth continuation with the surface of the connector (2, 2', 2'').

6. A connector according to any one of the preceding claims, characterised in that the first end (20, 20') comprises a tapered portion (21) at its forward end and a cylindrical portion (22) at its rear end, and that the or each barb (23, 24, 23', 24') is located on the cylindrical portion (22).

7. A connector according to any one of Claims 1 to 5, characterised in that the first end (20'') is tapered, and that the or each barb (23'', 24'') is located on the tapered end.

8. A connector according to any one of the preceding claims, characterised in that the forward end (40'') of first end (20'') is bevelled to facilitate insertion in a tube (1).

9. An assembly of a tube (1) and a connector (2, 2', 2'') according to any one of the preceding claims, characterised in that the first end (20, 20', 20'') of the connector is inserted within an end of the tube, and that the tube (1) is a tracheal tube.

10. An assembly of a tube (1) and a connector (2, 2', 2'') according to any one of Claims 1 to 8, characterised in that the first end (20, 20', 20'') of the connector is inserted within an end of the tube, and that the tube (1) is reinforced with a helical member (10).
